# EUROPEAN PATENT APPLICATION

(11) **EP 1 774 966 A1**
(43) Date of publication of application: **18.04.2007**
(21) Application number: 05766200.9
(22) Date of filing: 13.07.2005
(51) Int. Cl.: A61K 31/198, A23L 1/305, A61P 1/16, A61P 35/00

(54) **INHIBITOR FOR THE ONSET AND PROGRESS OF LIVER CANCER TO BE USED IN HEPATITIS C VIRUS-POSITIVE HUMAN LIVER CIRRHOSIS PATIENTS**

(30) Priority: 14.07.2004 JP 2004207693
(71) Applicant: Ajinomoto Co., Inc., Tokyo 104-8315 (JP)
(72) Inventor: KUMADA, Hiromitsu, 1580095 (JP); MUTO, Yasutoshi, Dai2 Orenji Hisaya 4B, Nagoya-shi, Aichi 4610001 (JP); SATO, Shunichi, 0200107 (JP); WATANABE, Akiharu, 7038267 (JP)
(74) Representative: Nicholls, Kathryn Margaret
(86) International application number: PCT/JP2005/013338
(87) International publication number: WO 2006/006729

(57) **Abstract**

The present invention provides, as a pharmaceutical agent having an effect to inhibit the development and/or progression of liver cancer in hepatitis C virus-positive human cirrhosis patients, an agent for inhibiting the development and/or progression of liver cancer in hepatitis C virus-positive human cirrhosis patients, which contains three kinds of amino acids of isoleucine, leucine and valine.

## Description

### Technical Field

The present invention relates to a pharmaceutical agent that characteristically inhibits the development or progression of liver cancer in hepatitis C virus-positive human cirrhosis patients.

### Background Art

The mechanism of the progress from hepatitis or liver cirrhosis to liver cancer has not been entirely elucidated. To inhibit liver cancer, however, it is considered important to remove the etiology of hepatitis or liver cirrhosis.

Based on the etiology, cirrhosis is divided into hepatitis C virus (HCV)-related cirrhosis (cirrhosis type C), hepatitis B virus (HBV)-related cirrhosis (cirrhosis type B), alcoholic cirrhosis, and others (non-B/non-C, special type and the like). Simultaneous infection with HCV and HBV may occur (HBV-HCV mixed type). Of these etiologies, the number of patients with cirrhosis caused by hepatitis C virus (hereinafter to be also referred to as hepatitis C virus-positive human cirrhosis patients) is the largest.

It has been reported that the liver carcinogenic rate of type C cirrhosis patients steadily increases with the lapse of time, unlike the type B cirrhosis patients (see K. Ikeda et al., "Hepatology" (1993) 18:47-53). It is also said that about 70 to 80% of liver cancer development is caused by infection with hepatitis C virus. In hepatitis C virus-positive human cirrhosis patients, therefore, inhibition of the development of liver cancer is important for improved prognosis and, as the situation stands, an effective agent for inhibiting the development or progression of liver cancer in hepatitis C virus-positive human cirrhosis patients is desired.

As a current treatment for cirrhosis, for example, it has been reported that removal of hepatitis virus by an interferon treatment significantly inhibits carcinogenesis (see Y. Imai et al., "Annals Internal Medicine" (1998) 129:94). As a method for removing hepatitis virus, a treatment using an antiviral drug can be mentioned (see J.G. McHutchison et al., "The New England Journal of Medicine" (1998)339:1485, and J. Main et al., "Journal of Viral Hepatitis" (1996)3:211).

In any event, it is not possible to remove the virus from all patients, and complete prophylaxis of liver cancer has not been achieved.

While attempts have been made to inhibit development of liver cancer by inhibiting chronic inflammation with a liver protecting agent and the like, carcinogenesis still cannot be prevented entirely (see H. Oka et al., "Cancer" (1995) 76:743, and Y. Arase et al., "Cancer" (1997) 79:1494).

Furthermore, while the treatment or inhibition of cancer by deficiency or excessive administration of particular amino acids, such as methionine deficiency, valine deficiency, aspartic acid deficiency, lysine deficiency, cysteine deficiency, phenylalanine deficiency, increased administration of arginine, increased administration of glutamine and the like, has been also tried, the situation is by no means satisfactory (see Tetsuro Nishihira et al., "The Japanese journal of parenteral and enteral nutrition (JJPEN)", (1997)19:195-199 and Norie Kurokawa et al., "Japanese Journal of Nutritional Assessment" (1992) vol. 9 No. 2 p. 142-146).

Meanwhile, some patients with cirrhosis accompany a decrease in the blood Fischer's ratio [branched chain amino acid mol (isoleucine + leucine + valine)/aromatic amino acid mol (phenylalanine + tyrosine)] and a decrease in the serum albumin concentration, which are caused by abnormality in the metabolism of protein and/or amino acid. The serum albumin concentration and Fischer's ratio in these cases show a positive correlation (see Yasutoshi Muto et al., "Japan Medical Journal" (1983) 3101:3), and lower serum albumin concentration is known to cause worsening of vital prognosis (see Yasutoshi Muto et al., "the Japanese journal of parenteral and enteral nutrition (JJPEN)" (1995) 17:208).

To improve hypoalbuminemia caused by these disorders of amino acid metabolism in patients with cirrhosis, administration of a branched chain amino acid (BCAA) combination preparation called LIVACT (registered trademark) has been employed.

As regards Aminoleban EN (registered trademark), which is a nutritional status improving drug for patients with chronic hepatic failure associated with hepatic encephalopathy, it has been reported that administration of a feed containing this drug to rat with chemical-induced liver cancer revealed a tendency to inhibit liver cancer as compared to a control group bred on a conventional feed (see Norie Kurokawa et al., Japanese Journal of Nutritional Assessment (1992) vol. 9 No. 2 p. 142-146). However, this reference does not at all contain description suggesting that such carcinogenesis inhibitory effect is related to a particular component in Aminoleban EN (registered trademark), which is a balanced nutrition product containing carbohydrates, protein and fat, supplemented with various vitamins and minerals.

Similarly, it has been reported that a long-term administration of a BCAA added feed to LEC rats (spontaneous carcinogenesis model) exhibited an inhibitory action on the progress of cancer, though the incidence of cancer did not vary from that of the control group (see Noriaki Okuse et al., "Acta Hepatologica Japonica" (2002) 43 Supplement(2): A359). However, this reference does not describe the kind of BCAA, mixing ratio and the like, nor does it suggest that such action is related to a particular component in BCAA.

The aforementioned LIVACT (registered trademark) is a preparation consisting of three kinds of branched chain amino acids of isoleucine, leucine and valine, which was developed for the purpose of correcting the Fischer's ratio, increasing serum albumin concentration and improving clinical conditions, by orally supplementing these branched chain amino acids at an appropriate ratio. However, its action to inhibit carcinogenesis is not known. The technical relationship between hypoalbuminemia and cancer development has not been known, either.

### Disclosure of the Invention

The problem to be solved by the present invention is provision of a pharmaceutical agent effective for inhibiting the development and/or progression of liver cancer in hepatitis C virus-positive human cirrhosis patients.

The present inventors have conducted intensive studies in an attempt to solve the aforementioned problems and found that a composition comprising three kinds of amino acids of isoleucine, leucine and valine as active ingredients has an inhibitory action on the development and/or progression of liver cancer in hepatitis C virus-positive human cirrhosis patients, which resulted in the completion of the present invention. Accordingly, the present invention encompasses the following items.
(1) An agent for inhibiting the development and/or progression of liver cancer in hepatitis C virus-positive human cirrhosis patients, which comprises three kinds of amino acids of isoleucine, leucine and valine.
(2) The agent of (1), wherein the cirrhosis patient is a male.
(3) The agent of (1) or (2), wherein the cirrhosis is decompensated cirrhosis.
(4) The agent of any one of (1) to (3), wherein the weight ratio of isoleucine, leucine and valine is 1:1.5 to 2.5:0.8 to 1.7.
(5) The agent of any one of (1) to (4), whose daily dose is 2.0 g to 50.0 g.
(6) A pharmaceutical composition for inhibiting the development or progression of liver cancer in hepatitis C virus-positive human cirrhosis patients, which comprises three kinds of amino acids of isoleucine, leucine and valine and a pharmaceutically acceptable carrier.
(7) The composition of (6), wherein the cirrhosis patient is a male.
(8) The composition of (6) or (7), wherein the cirrhosis is decompensated cirrhosis.
(9) The composition of any one of (6) to (8), wherein the weight ratio of isoleucine, leucine and valine is 1:1.5 to 2.5:0.8 to 1.7.
(10) The composition of any one of (6) to (9), whose daily dose in the total amount of isoleucine, leucine and valine is 2.0 g to 50.0 g.
(11) A method for the prophylaxis or treatment of liver cancer, which comprises administering effective amounts of three kinds of amino acids of isoleucine, leucine and valine to a hepatitis C virus-positive human cirrhosis patient.
(12) The method of (11), wherein the cirrhosis patient is a male.
(13) The method of (11) or (12), wherein the cirrhosis is decompensated cirrhosis.
(14) The method of any one of (11) to (13), wherein the weight ratio of isoleucine, leucine and valine is 1:1.5 to 2.5:0.8 to 1.7.
(15) The method of any one of (11) to (14), whose daily dose in the total amount of isoleucine, leucine and valine is 2.0 g to 50.0 g.
(16) Use of isoleucine, leucine and valine for the production of an agent for inhibiting the development and/or progression of liver cancer in hepatitis C virus-positive human cirrhosis patients, which comprises three kinds of amino acids of isoleucine, leucine and valine.
(17) The use of (16), wherein the cirrhosis patient is a male.
(18) The use of (16) or (17), wherein the cirrhosis is decompensated cirrhosis.
(19) The use of any one of (16) to (18), wherein the isoleucine, leucine and valine are used at a weight ratio of 1:1.5 to 2.5:0.8 to 1.7.
(20) The use of any one of (16) to (19), wherein a daily dose of the agent for inhibiting the development and/or progression of liver cancer comprising three kinds of amino acids of isoleucine, leucine and valine is 2.0 g to 50.0 g.
(21) A commercial package comprising an agent for inhibiting the development and/or progression of liver cancer in hepatitis C virus-positive human cirrhosis patients, which comprises three kinds of amino acids of isoleucine, leucine and valine, and a written matter containing an explanation on the use of the agent for inhibiting the development and/or progression of liver cancer.
(22) A food comprising three kinds of amino acids of isoleucine, leucine and valine, with an indication of use for inhibiting the development and/or progression of liver cancer in hepatitis C virus-positive human cirrhosis patients.

### Brief Description of the Drawings

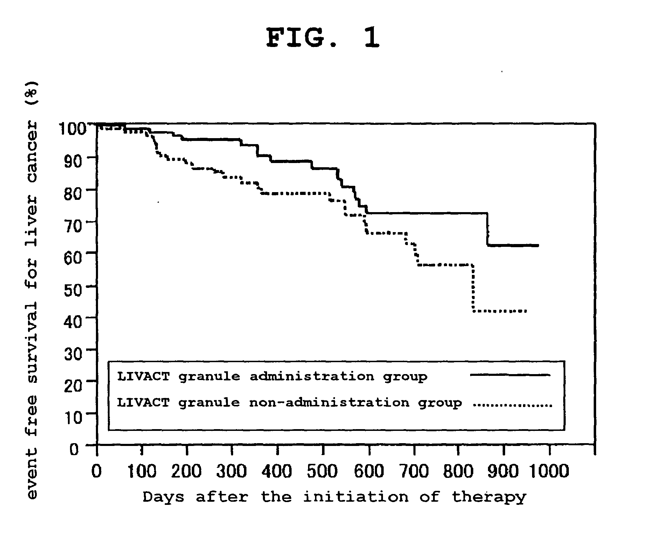

Fig. 1 shows the event free survival for liver cancer in HCV positive male cirrhosis patients subjected to dietary therapy and LIVACT granule administration.

### Best Mode for Embodying the Invention

The embodiment of the present invention is explained in the following.

The mode of administration and dosage form of the agent for inhibiting the development and/or progression of liver cancer in hepatitis C virus-positive human cirrhosis patients of the present invention (hereinafter to be referred to as the pharmaceutical agent of the present invention) may be oral administration or parenteral administration, and as an agent for oral administration, solids such as powder, granule, capsule, tablet, chewable agent and the like and liquids such as solution, syrup and the like can be mentioned, and as an agent for parenteral administration, injection, spray and the like can be mentioned.

The pharmaceutical agent of the present invention is useful for human, particularly male patients.

Referring to the pathology to which the pharmaceutical agent of the present invention is applied, it is effective for the prevention of liver cancer in hepatitis C virus-positive human cirrhosis patients, and inhibition of the development or progression of and the cure of liver cancer in hepatitis C virus-positive human cirrhosis patients, for which conventional therapeutic agents for hepatic diseases failed to show a sufficient treatment effect. Particularly, the agent is effective when cirrhosis is decompensated cirrhosis.

While a composition containing three kinds of amino acids of isoleucine, leucine and valine as active ingredients is known to improve hypoalbuminemia in cirrhosis patients, in the application of the inhibition of the development or progression of liver cancer in hepatitis C virus-positive human cirrhosis patients of the present invention, reduction of the serum albumin value is not necessarily the requirement.

It is known that the liver has a large functional reserve. For the cirrhosis patients, the disease stage when the functional reserve markedly decreases and gastrointestinal hemorrhage, ascites, encephalopathy, infection and the like appear is accorded a disease name, decompensated cirrhosis. On the other hand, the disease stage when the functional reserve has not decreased much is accorded a disease name, compensated cirrhosis.

Patients with cirrhosis type C, cirrhosis type B or mixed type can be identified by testing for infection with HCV and HBV in the cirrhosis patients. For infection with HCV or HBV, measurement of HBs antigen (RIA, EIA and the like) and measurement of HCV antibody (commercially available HCV antibody measurement kit) are performed. Since HCV antibody-negative cirrhosis patients also include those who test positive in the HCV-RNA measurement by PCR, additional measurement of HCV-RNA by PCR is useful for HCV antibody-negative patients. The cirrhosis patients confirmed to be positive by the above-mentioned method or other method capable of confirming HCV or HBV infection can be identified as HCV type, HBV type or mixed type cirrhosis patients. In other words, the hepatitis C virus-positive human cirrhosis patients in the present invention refer to human cirrhosis patients confirmed to have infected with HCV and hepatitis C-derived(related) cirrhosis patient.

The isoleucine, leucine and valine in the present invention may be D forms or L forms, with preference given to L forms.

The mixing ratio of the three kinds of amino acids is 1:1.5 to 2.5:0.8 to 1.7, particularly preferably 1:1.9 to 2.2:1.1 to 1.3, by weight ratio. When the ratio is outside this range, significant action and effect are difficult to achieve.

While the dose varies depending on the age, body weight, and condition of subject patients and administration method, as a rough measure, it generally includes isoleucine in an amount of 0.5 to 30.0 g, leucine in an amount of 1.0 to 60.0 g and valine in an amount of 0.5 to 30.0 g for one day. In the case of an ordinary adult, it preferably includes isoleucine in an amount of 2.0 to 10.0 g, leucine in an amount of 3.0 to 20.0 g and valine in an amount of 2.0 to 10.0 g, more preferably isoleucine in an amount of 2.5 to 3.5 g, leucine in an amount of 5.0 to 7.0 g and valine in an amount of 3.0 to 4.0 g, for one day. The total amount of the three amino acids is preferably about 2.0 g to 50.0 g per day, which is administered in 1 to 6, preferably 1 to 3, portions.

Isoleucine, leucine and valine, which are the active ingredients in the present invention, may be contained in a preparation individually or in any combination, or all may be contained in one kind of preparation. For administration after individual processing into preparations, the administration routes and the administration dosage forms thereof may be the same or different, and the timing of the administration may be simultaneous or separate, which can be appropriately determined based on the kind of pharmaceutical agents to be concurrently used and the effect thereof. That is, the agent for inhibiting the development and/or progression of liver cancer of the present invention may be a preparation simultaneously containing three kinds of amino acids, or take the form of concomitant drugs prepared separately and used in combination. The present invention encompasses all these forms. Particularly, a dosage form containing three kinds of amino acids in a single preparation is preferable, since it can be administered conveniently.

In the present invention, the "weight ratio" means a ratio of the weights of respective ingredients in the preparation. For example, when respective active ingredients of isoleucine, leucine and valine are contained in a single preparation, it means a ratio of individual contents, and when each of the active ingredients alone, or any combination thereof is/are contained in plural preparations, it means a ratio of the weight of each active ingredient contained in each preparation.

In the present invention, the ratio of actual dose shows a ratio of a single dose or a daily dose of each active ingredient per one subject of administration (i.e., patient). For example, when each active ingredient of isoleucine, leucine and valine is contained in a single preparation and administered to a subject of administration, the weight ratio corresponds to the dose ratio. When each active ingredient is contained separately or in any combination thereof in plural preparations and administered, the weight ratio corresponds to a ratio of the total amount of each active ingredient in each preparation administered at one time or in one day.

Isoleucine, leucine and valine have been widely used in the fields of pharmaceutical agents and food, and their safety has been established. For example, acute toxicity (LD₅₀) of the pharmaceutical composition of the present invention containing these amino acids at a ratio of 1:2:1.2 is not less than 10 g/Kg when orally administered to mice.

The pharmaceutical agent of the present invention can be formulated into solids such as powder, granule, capsule, tablet, chewable and the like, liquids such as solution, syrup and the like, or, injection, spray and the like by conventional methods.

Where necessary for preparation, appropriate pharmacologically acceptable carriers, such as excipient, binder, lubricant, solvent, disintegrant, dissolution aids, suspending agent, emulsifier, isotonicity agent, stabilizer, soothing agent, preservative, antioxidant, corrigent, coloring agent and the like are mixed to give preparations.

As the excipient, organic excipients such as saccharides (e.g. lactose, glucose, D-mannitol etc.), starches, celluloses (e.g. crystalline cellulose etc.), and the like, inorganic excipients such as calcium carbonate, kaolin and the like, and the like can be mentioned; as the binder, gelatinized starch, gelatin, gum arabic, methylcellulose, carboxymethylcellulose, sodium carboxymethylcellulose, crystalline cellulose, D-mannitol, trehalose, hydroxypropylcellulose, hydroxypropylmethylcellulose, polyvinylpyrrolidone, polyvinyl alcohol and the like can be mentioned; as the lubricant, fatty acid salts such as stearic acid, stearate and the like, talc, silicates and the like can be mentioned; as the solvent, purified water, saline and the like can be mentioned: as the disintegrant, low substituted hydroxypropylcellulose, chemically modified cellulose, starches and the like can be mentioned; as the dissolution aids, polyethylene glycol, propylene glycol, trehalose, benzyl benzoate, ethanol, sodium carbonate, sodium citrate, sodium salicylate, sodium acetate and the like can be mentioned; as the suspending agent or emulsifier, sodium lauryl sulfate, gum arabic, gelatin, lecithin, glyceryl monostearate, polyvinyl alcohol, polyvinyl pyrrolidone, celluloses such as sodium carboxymethylcellulose and the like, polysorbates, hydrogenated polyoxyethylene castor oil and the like can be mentioned; as the isotonicity agent, sodium chloride, potassium chloride, saccharide, glycerine, urea and the like can be mentioned; as the stabilizer, polyethylene glycol, sodium dextran sulfate, other amino acids and the like can be mentioned; as the soothing agent, glucose, calcium gluconate, procain hydrochloride and the like can be mentioned; as the preservative, paraoxybenzoic esters, chlorobutanol, benzyl alcohol, phenethyl alcohol, dehydroacetic acid, sorbic acid and the like can be mentioned; as the antioxidant, subsulfate, ascorbic acid and the like can be mentioned; as the corrigent, sweetener, flavoring and the like conventionally used in the fields of pharmaceutical agents and food can be mentioned; and as the coloring agent, artificial color conventionally used in the fields of pharmaceutical agents and food can be mentioned.

The pharmaceutical agent of the present invention may contain other therapeutic drugs for liver diseases, such as interferon, glycyrrhizin, ursodeoxycholic acid, ribavirin, Chinese medicine sho-saiko-to and the like.

These preparations can be administered by any administration method such as oral, injection or topical administration and the like.

The present invention is superior in safety since the active ingredient is amino acid, and can be conveniently used even in the form of a food or drink. The present invention does not pose any particular difficulty in application to food and drink and, for example, it can be consumed in admixture with juice, milk, confectionery, jelly and the like. It is also possible to provide such food as a food with health claims, and the food with health claims includes food and drink with an indication of use for the inhibition of the development or progression of liver cancer in hepatitis C virus-positive human cirrhosis patients, particularly, Food for Specified Health Use and the like.

In a package comprising the pharmaceutical agent of the present invention and a written matter containing an explanation on the use thereof, examples of the written matter include what is called a package insert containing an explanation relating to use, efficacy, administration method etc., and the like.

### Example

The present invention is explained in more detail by referring to Example in the following. However, the following Example should be considered as an aid to obtain concrete understanding of the present invention, and the scope of the present invention is not at all limited by the following Example.

### Example 1

Inhibition of liver cancer in hepatitis C virus-positive human cirrhosis patients of the present invention was examined.

The effect of LIVACT administration on dietary therapy was examined in decompensated cirrhosis patients in the age group of from 20 to 75 years old, who were then suffering from or had a history of ascites, edema or hepatic encephalopathy, and all showed a serum albumin value of not more than 3.5 g/dl without administration of an albumin preparation. The infecting virus of the decompensated cirrhosis patients was identified by a conventional test method, such as HCV antibody measurement kit, HCV-RNA measurement and the like.

For the dietary therapy group, only the dietary therapy was applied, where the dietary instruction was given to prevent shortage of protein amount. The basic therapy of the LIVACT administration group was a dietary therapy and one package per dose of a branched chain amino acid preparation LIVACT Granules (trademark) (Ajinomoto Co., Inc., weight ratio of isoleucine, leucine, valine, 1:2:1.2 (isoleucine: 0.952 g, leucine: 1.904 g, valine: 1.144 g)) was administered orally 3 times daily after meal.

The development of liver cancer was examined in 88 cases of the dietary therapy group and 94 cases of the LIVACT administration group, both of HCV positive male cirrhosis patients, by image analysis, cytologic diagnosis and the like. As a result (Fig. 1), the development of liver cancer was observed in 25 cases of the dietary therapy group but 17 cases of the LIVACT administration group during the test period, and the development was significantly inhibited (Wilcoxon p=0.0489). In the Figure, the straight line shows a LIVACT Granule administration group, and the dotted line shows a dietary therapy group (LIVACT Granule non-administration group).

### Industrial Applicability

The agent for inhibiting the development or progression of liver cancer in hepatitis C virus-positive human cirrhosis patients provided by the present invention, which contains three kinds of branched chain amino acids of isoleucine, leucine and valine inhibits the development or progression of liver cancer in hepatitis C virus-positive human cirrhosis patients. Particularly, the agent is effective for male hepatitis C virus-positive human cirrhosis patients. In addition, it is particularly effective when cirrhosis is decompensated cirrhosis. Moreover, since the pharmaceutical composition of the present invention contains amino acids as active ingredients, it is highly safe and almost free of side effects, and can be administered for a long time. Therefore, the composition can be advantageously used for the prophylaxis or treatment over a long period up to the development of liver cancer.

The present application is based on a patent application No. 2004-207693 filed in Japan, the contents of which are hereby incorporated by reference.

## Claims

1. An agent for inhibiting the development and/or progression of liver cancer in hepatitis C virus-positive human cirrhosis patients, which comprises three kinds of amino acids of isoleucine, leucine and valine.

2. The agent of claim 1, wherein the cirrhosis patient is a male.

3. The agent of claim 1 or 2, wherein the cirrhosis is decompensated cirrhosis.

4. The agent of any one of claims 1 to 3, wherein the weight ratio of isoleucine, leucine and valine is 1:1.5 to 2.5:0.8 to 1.7.

5. The agent of any one of claims 1 to 4, whose daily dose is 2.0 g to 50.0 g.

6. A pharmaceutical composition for inhibiting the development or progression of liver cancer in hepatitis C virus-positive human cirrhosis patients, which comprises three kinds of amino acids of isoleucine, leucine and valine and a pharmaceutically acceptable carrier.

7. The composition of claim 6, wherein the cirrhosis patient is a male.

8. The composition of claim 6 or 7, wherein the cirrhosis is decompensated cirrhosis.

9. The composition of any one of claims 6 to 8, wherein the weight ratio of isoleucine, leucine and valine is 1:1.5 to 2.5:0.8 to 1.7.

10. The composition of any one of claims 6 to 9, whose daily dose in the total amount of isoleucine, leucine and valine is 2.0 g to 50.0 g.

11. A method for the prophylaxis or treatment of liver cancer, which comprises administering effective amounts of three kinds of amino acids of isoleucine, leucine and valine to a hepatitis C virus-positive human cirrhosis patient.

12. The method of claim 11, wherein the cirrhosis patient is a male.

13. The method of claim 11 or 12, wherein the cirrhosis is decompensated cirrhosis.

14. The method of any one of claims 11 to 13, wherein the weight ratio of isoleucine, leucine and valine is 1:1.5 to 2.5:0.8 to 1.7.

15. The method of any one of claims 11 to 14, whose daily dose in the total amount of isoleucine, leucine and valine is 2.0 g to 50.0 g.

16. Use of isoleucine, leucine and valine for the production of an agent for inhibiting the development and/or progression of liver cancer in hepatitis C virus-positive human cirrhosis patients, which comprises three kinds of amino acids of isoleucine, leucine and valine.

17. The use of claim 16, wherein the cirrhosis patient is a male.

18. The use of claim 16 or 17, wherein the cirrhosis is decompensated cirrhosis.

19. The use of any one of claims 16 to 18, wherein the isoleucine, leucine and valine are used at a weight ratio of 1:1.5 to 2.5:0.8 to 1.7.

20. The use of any one of claims 16 to 19, wherein a daily dose of the agent for inhibiting the development and/or progression of liver cancer comprising three kinds of amino acids of isoleucine, leucine and valine is 2.0 g to 50.0 g.

21. A commercial package comprising an agent for inhibiting the development and/or progression of liver cancer in hepatitis C virus-positive human cirrhosis patients, which comprises three kinds of amino acids of isoleucine, leucine and valine, and a written matter containing an explanation on the use of the agent for inhibiting the development and/or progression of liver cancer.

22. A food comprising three kinds of amino acids of isoleucine, leucine and valine, with an indication of use for inhibiting the development and/or progression of liver cancer in hepatitis C virus-positive human cirrhosis patients.
